# EUROPEAN PATENT SPECIFICATION

(11) **EP 4 247 334 B1**
(45) Date of publication and mention of the grant of the patent: **09.10.2024**
(21) Application number: 21815631.3
(22) Date of filing: 17.11.2021
(51) Int. Cl.: A61K 9/00, A61K 31/19, A61K 31/715, A61K 45/06, A61P 15/02, A61P 31/04, A61P 31/10

(54) **PHARMACEUTICAL COMPOSITION FOR USE IN THE PREVENTION OR TREATMENT OF A CONDITION ASSOCIATED WITH A REDUCTION IN THE NUMBER OF LACTOBACILLI IN THE VAGINA**
PHARMAZEUTISCHE ZUSAMMENSETZUNG ZUR VERWENDUNG ZUR PRÄVENTION ODER BEHANDLUNG EINES ZUSTANDES, DER MIT EINER REDUZIERUNG DER LACTOBACILLI-MENGE IN DER SCHEIDE VERBUNDEN IST
COMPOSITION PHARMACEUTIQUE À UTILISER DANS LA PRÉVENTION OU LE TRAITEMENT D'UNE CONDITION ASSOCIÉE À UNE RÉDUCTION DE LA QUANTITÉ DE LACTOBACILLES DANS LE VAGIN

(30) Priority: 17.11.2020 GB 202018068
(43) Date of publication of application: 27.09.2023
(73) Proprietor: UCL Business Ltd, London WC1E 6BT (GB)
(72) Inventor: LONG, Paul, London, The Strand, London WC2R 2LS (GB); SUTCLIFFE, Alastair, London W1T 4TP (GB); TULEU, Catherine, London W1T 4TP (GB)
(74) Representative: D Young & Co LLP
(86) International application number: PCT/GB2021/052973
(87) International publication number: WO 2022/106819

(56) References cited:
- EP-A1- 2 130 531
- US-A- 5 622 927
- US-B1- 6 440 949

## Description

### FIELD OF THE INVENTION

The present invention relates to a pharmaceutical composition comprising glycogen for use in preventing or treating conditions associated with a reduction in the number of Lactobacilli in the vagina of a subject relative to a healthy subject, for example vaginal infection or vaginal inflammation such as bacterial vaginosis, vaginitis, vulvovaginal candidiasis or Group B *Streptococcus* infection, or a method for the prevention or treatment of such a condition or resultant sequalae thereof with the composition.

### BACKGROUND TO THE INVENTION

Bacterial vaginosis (BV) is the most common vaginal infection in women of reproductive age worldwide, and is associated with significant adverse consequences including an increased risk of late miscarriage or preterm birth (1, 2), post-partum endometritis (3) and also a greater chance of acquiring sexually transmitted diseases, such as HIV (4). Reported prevalence rates range from 10-40% at any one time, depending upon the population studied. However, suboptimal methods of diagnosis and a high percentage of asymptomatic patients suggest the true prevalence of BV maybe very much higher (5). BV is associated with an increased volume of vaginal discharge having a foul, fishy odor.

The exact cause of BV is still unknown in the vast majority of instances, but alterations of both local host immunity and the genital tract microflora appear to contribute to the pathogenesis of BV (6). Under normal conditions, *Lactobacillus* bacteria are predominant in the vagina and are believed to control the growth of other microorganisms by producing hydrogen peroxide and lactic acid from vaginal glycogen to maintain vaginal acidity between pH 4 and 5. *Lactobacillus* is a genus of Gram-positive, facultative anaerobic or microaerophilic, rod-shaped, non-spore-forming bacteria.

In women with BV, however, normal vaginal lactobacilli are replaced by an overgrowth of other anaerobes with a concomitant decrease in lactobacilli numbers. Recent data suggest a primary role for *Gardnerella vaginalis* as a specific and perhaps sexually transmitted aetiological agent in BV (7-9), as was initially postulated by Gardner and Dukes in 1955 (10). Thus, while acid producing lactobacilli can be found in women with BV (11), their numbers may not be sufficient to overcome *Gardnerella. vaginalis,* which in turn replaces lactobacilli as the dominant microflora as the vaginal pH increases to around 7.8-8.2 (12). Conversely, numerous studies have shown that even when there is not a significant reduction in lactobacilli numbers, small decreases can be sufficient to allow overgrowth by yeast such as species of *Candida,* which cause a drop in pH below 4 consistent with symptom of vulvovaginal candidiasis ((27)-(32)).

Often women who experience the symptoms of BV complain of a foul, fishy odour and excessive vaginal discharge sufficiently unpleasant, that these women seek medical treatment. Traditional treatments have classically centred on the prescription-only antibiotics metronidazole and clindamycin. Metronidazole as a 7-day oral treatment has an 80-90 % cure rate after 1 month. Side effects include nausea, abdominal cramps and a metallic taste. The patient must refrain from alcohol intake, as it may produce antabuse effects. It is not recommended in the first trimester of pregnancy. Metronidazole is also available in vaginal gels, for example, METROGEL VAGINAL^{®}, yet despite their common use, vaginal gels are less than ideal. To be effective, the gels must be applied once or twice a day for a period of five days, usually at night. Clindamycin as a 7-day oral treatment has equal effects as metronidazole, and its side effects are less, although diarrhoea is possible and concerns about *Clostridium difficile* colitis have prevented widespread use (13, 14). Despite a high cure rate, a significant proportion of women suffer relapses and recurrences of BV, regardless whether the proceeding treatment was oral or intravaginal (15, 16). For example, a double-blind, placebo-controlled crossover trial has shown that intravaginal treatment with a 0.75 % (w/v) metronidazole gel resulted in a recurrence rate of about 15 % one month following treatment (17).

Antibiotics are a temporary treatment for BV. Antibiotics can eliminate the bacteria that cause BV, but at the same time also disrupt the natural balance of bacterial flora in the vagina, which can be disruptive in the long term leading to other infections including candidiasis.

Canesbalance^{®} and Balance Activ^{®} are commercial products which claim to reduce the symptoms of BV. These formulations are composed of lactic acid and glycogen. Because these formulations are acidic, they can cause irritation and even bleeding. The acidity of these formulations is to affect a rapid drop in the vaginal pH, reducing overgrowth of pathogenic bacteria and to promote regrowth of acid tolerant lactobacilli. These formulations, therefore, are not a cure and only offer at best, some temporary relief in some women. This regrowth will be only of the most acid tolerant and fast growing lactobacilli serotypes (perhaps dominated by a single or just a few serotypes), which may not persist overtime, resulting in replacement with the pathogenic overgrowth and recurrence of symptoms.

Many healthy women consider the symptoms of BV to indicate a lack of proper hygiene, rather than a medical problem and often use douches purchased without a prescription, rather than seek medical advice. The idea of washing out the foul smelling discharge with an acidic douche may have a simplistic appeal. Medically, however, douching is discouraged as studies have demonstrated an association between douching and Pelvic Inflammatory Disease (PID), ectopic pregnancy, tubal infertility, and reduced fertility (18-21). Different alternative measures have also been advised, including live yoghurt or *Lactobacillus acidophilus* preparations, although studies to date have not yet demonstrated benefits from the use of probiotics (15). In U.S. Patent No. 6,440,949 the use of one or more saccharides (but not glycogen) in acidic prebiotic formulations is suggested as a method to decrease vaginal pH. In a Danish based clinical trial, the patent tested different concentrations of saccharides, but none of the concentrations showed a significant decrease in pH and, furthermore, none of the concentrations eliminated odor causing bacteria.

EP1072269B1 discloses the use of glycogen (alone at a concentration of between 2.5% and 17 w/v % and together with other polysaccharides) in a lactic acid gel as a treatment for BV; although clinical benefits were reported to be poor.

Therefore, in view of the fact that BV is currently the most prevalent form of vaginal infection in women of reproductive age, there is a real and immediate need for new compositions that address the shortcomings of currently available BV treatments. For example, it would be desirable to have available an intravaginal treatment that reduces the rate of recurrence of BV following a successful course of treatment; with additional benefits of reducing gynaecological complications such as preterm delivery. There is also a need to provide new treatments for other vaginal infections and inflammation.

### SUMMARY OF THE INVENTION

In an aspect of the invention, provided is a pharmaceutical composition for use in the prevention or treatment of a condition associated with a reduction in the number of Lactobacilli in the vagina of a subject relative to a healthy subject, wherein the composition comprises from 1.3 to 1.7 w/v % glycogen and less than 0.1 w/v % lactic acid.

The inventors have surprisingly found that the composition of the invention causes a significant increase in the number of Lactobacilli in the vagina of a treated subject, leading to the effective treatment of a number of vaginal infections and inflammations, for example bacterial vaginosis, vaginitis, vulvovaginal candidiasis or Group B *Streptococcus.*

In a particularly preferred embodiment the composition contains no lactic acid, i.e. the composition contains 0 w/v% lactic acid.

In a preferred embodiment the pharmaceutical composition further comprises water and a cellulose derivative, optionally from 94 to 97 w/v % water and from 2 to 5 w/v % of a cellulose derivative. Typically the pharmaceutical composition comprises from 1.3 to 1.7 w/v % glycogen, optionally from 1.4 to 1.6 w/v % glycogen, water and a cellulose derivative. Typically the pharmaceutical composition comprises from 1.3 to 1.7 w/v % glycogen, optionally from 1.4 to 1.6 w/v % glycogen, and an aqueous hydrogel comprising a cellulose derivative.

In a particularly preferred embodiment the pharmaceutical composition comprises about 1.5 w/v % glycogen.

Providing the correct amount of glycogen in the pharmaceutical composition is particularly important to achieve the therapeutic effect.

Without being bound by theory it is understood that a pharmaceutical composition comprising 1.3 to 1.7 w/v %, preferably 1.4 to 1.6 w/v %, most preferably about 1.5 w/v % glycogen, provides an optimum concentration of glycogen such that it acts as a carbon source for Lactobacilli, aiding in their growth. At lower concentrations, for example 1 w/v % glycogen, the concentration of carbon is not sufficient to support good Lactobacillus growth. Higher concentrations, for example 2 w/v % glycogen, or 2.5 to 17 w/v% as disclosed in EP1072269B1 can actually inhibit bacterial growth. The inhibition of bacterial growth at high saccharide concentrations is well known, and saccharides can be used as preservatives, for example in jams.

Typically, the glycogen is obtainable from an animal source, optionally wherein the glycogen is oyster, mussel or bovine glycogen.

Typically the cellulose derivative is a cellulose ether. The cellulose ether may be methylcellulose (MC), hydroxypropyl methylcellulose (HPMC), ethyl cellulose (EC), hydroxyethyl cellulose (HEC), sodium carboxymethylcellulose (NaCMC) , or combinations thereof. Preferably the cellulose ether is hydroxypropyl methylcellulose.

The pH of the pharmaceutical composition may be from 5.7 to 6.7. Preferably the pH of the pharmaceutical composition is from 6.0 to 6.2.

Typically the pharmaceutical product is in the form of a hydrogel.

The pharmaceutical composition may be a vaginal pessary.

The pharmaceutical composition may be mucoadhesive, for example it may comprise a mucoadhesive film or gel or mucoadhesive *in situ* gelling liquid crystalline precursor system.

The pharmaceutical composition may further comprise one or more pharmaceutically acceptable excipients.

The pharmaceutical composition may further comprise one or more additional therapeutic agents. The one or more additional therapeutic agents may be selected from an antibiotic, an antibacterial agent, an antifungal agent, an antiparasitic agent, an antiviral agent, an antiseptic agent and an anti-inflammatory agent.

In a further aspect, the present invention provides a method for the prevention or treatment of a condition associated with a reduction in the number of Lactobacilli in the vagina of a subject relative to a healthy subject, the method comprising the step of administering to a subject in need thereof a pharmaceutical composition as defined above.

In an embodiment of the pharmaceutical composition for use or method of the invention the use or method comprises providing an increase in the number of Lactobacilli in the vagina of the subject relative to an untreated subject with the condition. In an embodiment of the pharmaceutical composition for use or method of the invention the use or method comprises restoring the number of Lactobacilli in the vagina to a normal level i.e. the individualized nature of the vaginal microbiome makes the response to the pharmaceutical composition subject-specific affording each patient their own 'Personalized microbiome therapy'. The Lactobacilli may be endogenous Lactobacilli, i.e. normally present in the vagina of a subject who is healthy. The Lactobacilli may comprise *Lactobacillus gasseri, Lactobacillus acidophilus, Lactobacillus paracasei, Lactobacillus salivarius, Lactobacillus johnsonii* or combinations thereof. Fermentation of glycogen as a sole carbon source by Lactobacilli not only provides nutrition allowing restoration of Lactobacilli to a normal level, but the end products of the fermentation (lactic acid, hydrogen peroxide) cause a restoration in pH. In the case of bacterial vaginosis, the pH drops from alkaline to slightly acidic. Lactic acid has been widely demonstrated to inhibit growth of *Candida* species that cause vulvovaginal candidiasis. Therefore, a restoration in Lactobacilli numbers increases the concentration of lactic acid in the vagina, inhibiting the growth of pathogenic *Candida* species (33).

The condition to be prevented or treated is typically a vaginal infection, vaginal inflammation, or complications therefrom, optionally a recurrent vaginal infection or vaginal inflammation. The complication may be preterm birth, stillbirth, postpartum vaginal infection or vaginal inflammation.

In preferred embodiments of the invention the condition to be prevented or treated is bacterial vaginosis, vaginitis, vulvovaginal candidiasis or Group B *Streptococcus* infection.

In one embodiment the subject to be treated is pregnant.

In a preferred embodiment the condition to be prevented or treated is Group B *Streptococcus* infection and the subject is pregnant.

In embodiments of the composition for use or method of the invention the condition to be prevented or treated is associated with an increase in the number of pathogenic bacteria or yeast in the vagina of a subject relative to a healthy subject, optionally Gardnella vaginalis or Group B Steptococcus bacteria or a species of Candida yeast, optionally Candida albicans.

Typically the pharmaceutical composition is suitable for intravaginal administration.

### DESCRIPTION OF THE FIGURES

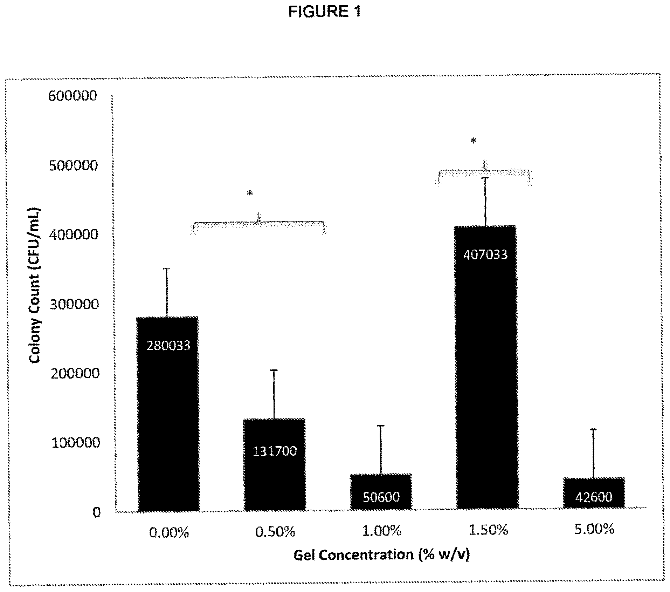

Figure 1 shows average colony counts for a range of glycogen concentrations from Patient 1. Eight colonies from the patient were each grown in a medium comprising 0% w/v glycogen for 72 hours. 10 microlitres of this solution was then incubated for 96 hours in each glycogen concentration and subsequently plated onto Lactobacillus selective agar in triplicate n = 24. Error bars show the deviation from the true value (mean) with samples showing significance marked with an asterisk (*).

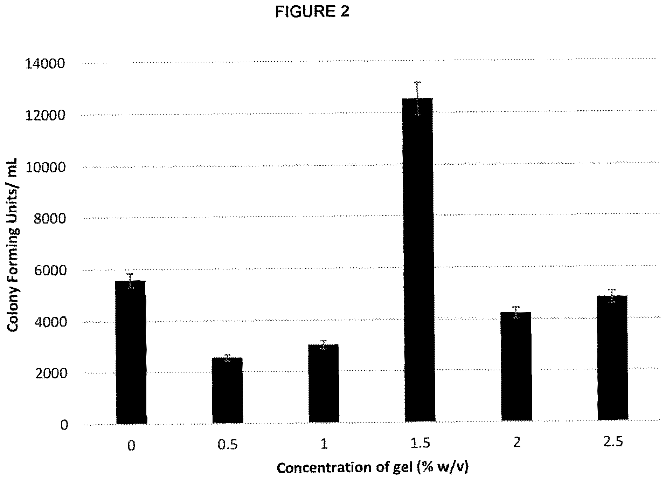

Figure 2 shows average colony counts for a range of glycogen concentrations from Patient 2. Three colonies from the patient were each grown in a medium comprising 0% w/v glycogen for 72 hours. 10 microlitres of this solution was then incubated for 72 hours in each glycogen concentration and subsequently plated onto Lactobacillus selective agar in triplicate n = 9.

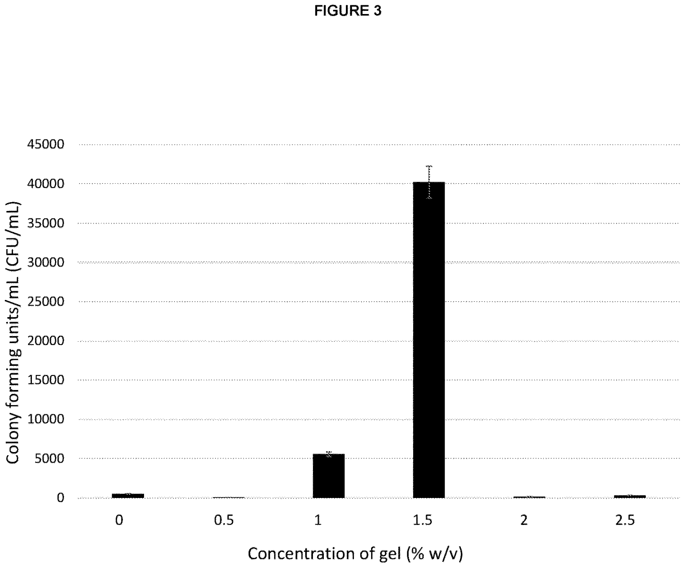

Figure 3 shows average colony counts for a range of glycogen concentrations from Patient 3. Three colonies from the patient were each grown in a medium comprising 0% w/v glycogen for 72 hours. 10 microlitres of this solution was then incubated for 72 hours in each glycogen concentration and subsequently plated onto Lactobacillus selective agar in triplicate n = 9.

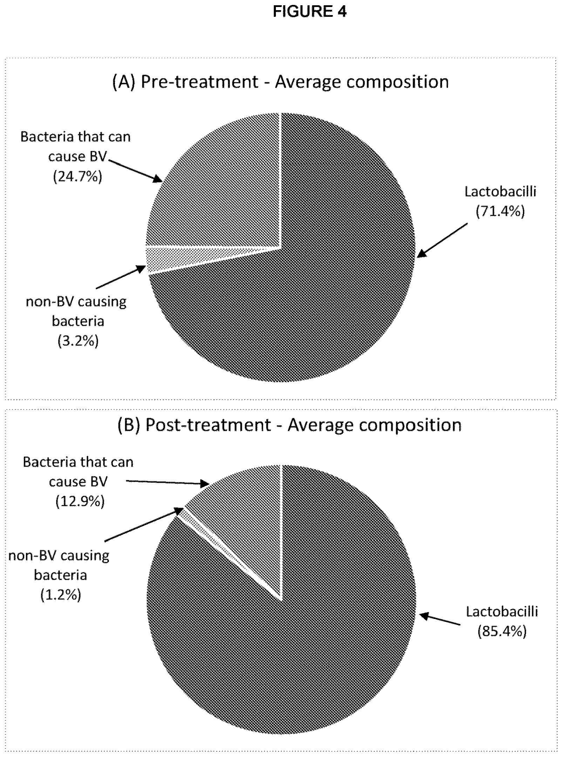

Figure 4 shows a pie chart of the relative proportions of bacterial species in the vagina of a patient (A) before treatment with the composition of the invention and (B) after treatment with the composition of the invention as determined by 16 S rRNA amplicon sequencing.

### DETAILED DESCRIPTION

In an aspect of the invention, provided is a pharmaceutical composition for use in the prevention or treatment of a condition associated with a reduction in the number of Lactobacilli in the vagina of a subject relative to a healthy subject, wherein the composition comprises from 1.3 to 1.7 w/v % glycogen and less than 0.1 w/v % lactic acid.

A skilled person would be aware of a number of conditions associated with a reduction in the number of Lactobacilli in the vagina of a subject relative to a healthy subject, for example vaginal infection or vaginal inflammation, in particular bacterial vaginosis, vaginitis, or Group B *Streptococcus* infection.

A skilled person would also know how to measure the number of Lactobacilli in the vagina of a subject. For example, a swab may be taken from the vagina of the subject, and used to inoculate a medium suitable for growth of Lactobacilli. After incubation, the grown bacteria are then cultured onto Lactobacilli specific agar plates, and incubated anaerobically at 37 °C for a suitable period of time. The number of bacterial colonies is then counted. The number of Lactobacilli is typically expressed in colony-forming units (CFU). Lactobacilli are the most prevalent and often numerically dominant microorganisms in the vagina, at 10⁷ -10⁸ CFU mL-¹ of vaginal fluid in healthy premenopausal women (see Farage MA, Miller KW, Sobel JD (2010) Dynamics of the vaginal ecosystem-hormonal influences. Infect Dis Res Treat 3:1-15, and Boris S, Barbés C (2000) Role played by lactobacilli in controlling the population of vaginal pathogens. Microbes Infect 2:543-546). CFU is a unit used to estimate the number of viable bacteria in a sample. Viable is defined as the ability to multiply via binary fission under the controlled conditions. Counting with colony-forming units requires culturing the microbes and counts only viable cells, in contrast with microscopic examination which counts all cells, living or dead. The visual appearance of a colony in a cell culture requires significant growth, and when counting colonies it is uncertain if the colony arose from one cell or a group of cells. Expressing results as colony-forming units reflects this uncertainty. Typically, the pharmaceutical composition of the invention treats vaginal conditions wherein the CFU for vaginal Lactobacilli is significantly reduced relative to healthy subjects. The pharmaceutical composition of the invention causes a significant increase in the number vaginal Lactobacilli, i.e. a significant increase in the Lactobacilli CFU relative to untreated subjects suffering from the vaginal condition.

Thus in a preferred embodiment the present invention relates to a pharmaceutical composition for use in the prevention or treatment of vaginal infection or vaginal inflammation, optionally bacterial vaginosis, vaginitis, or Group B *Streptococcus* infection, wherein the composition comprises from 1.3 to 1.7 w/v % glycogen and less than 0.1 w/v % lactic acid.

The inventors have surprisingly found that the composition of the invention causes a significant increase in the number of Lactobacilli in the vagina of a treated subject, leading to the effective treatment of a number of vaginal infections and inflammations, for example bacterial vaginosis or vaginitis. It is understood that Lactobacilli on the vaginal epithelial surface use glycogen as a food source, producing lactic acid which keeps the environment at a lower pH and deters other bacterial types. The glycogen content of human vaginal tissue is discussed in Gregoire, A. T. et al, Fertility and Sterility, Vol. 22, No. 1, January 1971. However, whilst compositions comprising glycogen are disclosed in the prior art for the treatment of vaginal infections such as BV by altering the microflora of the vagina, glycogen is typically used in combination with another active ingredient such as lactic acid or another saccharide, or glycogen is used at a significantly higher concentration.

Providing the correct amount of glycogen in the pharmaceutical composition is particularly important to achieve the therapeutic effect.

Without being bound by theory it is understood that a pharmaceutical composition comprising 1.3 to 1.7 w/v %, preferably 1.4 to 1.6 w/v %, most preferably about 1.5 w/v % glycogen, provides an optimum concentration of glycogen such that it acts as a carbon source for Lactobacilli, aiding in their growth. At lower concentrations, for example 1 w/v % glycogen, the concentration of carbon is not sufficient to support good *Lactobacillus* growth. Higher concentrations, e.g. 2 w/v % glycogen, or 2.5 to 17 w/v% as disclosed in EP1072269B1 can actually inhibit bacterial growth. The inhibition of bacterial growth at high saccharide concentrations is well known, and saccharides can be used as preservatives, for example in jams.

Typically, the glycogen is obtainable from an animal source, optionally wherein the glycogen is oyster, mussel or bovine glycogen.

In a preferred embodiment the pharmaceutical composition further comprises water and a cellulose derivative, optionally from 94 to 97 w/v % water and from 2 to 5 w/v % of a cellulose derivative.

Typically the pharmaceutical composition comprises or consists of from 1.3 to 1.7 w/v % glycogen, optionally 1.4 to 1.6 w/v % glycogen, water and a cellulose derivative. For example, preferably the pharmaceutical composition comprises or consists of from 1.3 to 1.7 w/v % glycogen, optionally 1.4 to 1.6 w/v % glycogen, and an aqueous hydrogel comprising a cellulose derivative.

In a particularly preferred embodiment the pharmaceutical composition comprises about 1.5 w/v % glycogen.

A key advantage of the present invention is that the pharmaceutical composition can be formed, and can achieve a therapeutic effect using only three key components: glycogen, a cellulose derivative and water, although of course other components may be present. This simple composition is likely to provide fewer side effects, for example irritation and bleeding, than the compositions of the prior art.

The pharmaceutical composition comprises less than 0.1 w/v % lactic acid, optionally less than 0.05 w/v % lactic acid. The composition may contain trace amounts of lactic acid, but in the most preferred embodiment the composition contains no lactic acid, i.e. it contains 0 w/v % lactic acid. Prior art compositions comprising lactic acid for treating BV are known, for example compositions described in EP1072269B1, and commercially available Canesbalance^{®} and Balance Active ^{®} formulations. Lactic acid is included in these formulations to decrease the pH of the composition. When administered to patients these formulations affect a rapid drop in vaginal pH, reducing overgrowth of pathogenic bacteria and promoting regrowth of acid tolerant Lactobacilli. However, these highly acidic lactic acid-containing compositions can cause irritation and bleeding in patients. In addition, the lactic-acid containing compositions typically only enable regrowth of the most acid tolerant and fast growing Lactobacilli serotypes, which may not persist over time, resulting in replacement with pathogenic bacteria and recurrence of symptoms.

The composition of the invention is not highly acidic, and does not comprise more than trace quantities of lactic acid, and preferably is free from lactic acid, avoiding irritation and bleeding caused by the acidic formulations of the prior art.

In addition, the pharmaceutical composition of the invention has superior stability and shelf life than *Lactobacillus* preparations of the prior art.

The glycogen may be obtainable from a marine organism, for example an oyster. The glycogen may be Type II glycogen. Glycogen from a marine organism source is commercially available, for example Type II glycogen from oyster is available from Sigma Aldrich, catalogue number G8751. The glycogen may also be from other marine (such as varieties of mussels) or terrestrial animal sources (including mammalian muscle or liver), but not a plant source. Glycogen is a multi-branched polysaccharide of glucose that serves as a form of energy storage in bacteria (as well as in humans, animals, and fungi). Thus it is understood that the glycogen provided in the compositions of the invention provide an energy or carbon source for Lactobacilli, enabling them to thrive. Glycogen is a branched biopolymer consisting of linear chains of glucose residues with an average chain length of approximately 8-12 glucose units. Glucose units are linked together linearly by α(1→4) glycosidic bonds from one glucose to the next. Branches are linked to the chains from which they are branching off by α(1→6) glycosidic bonds between the first glucose of the new branch and a glucose on the stem chain.

The cellulose derivative may be any polymer comprising cellulose. Typically the cellulose derivative is a cellulose ether. The cellulose ether may be methylcellulose (MC), hydroxypropyl methylcellulose (HPMC), ethyl cellulose (EC), hydroxyethyl cellulose (HEC), sodium carboxymethylcellulose (NaCMC), or combinations thereof. Preferably the cellulose ether is hydroxypropyl methylcellulose. A combination of hydroxypropyl methyl cellulose and methylcellulose may also be used. Cellulose ethers are commercially available, for example Metalose ^{®} 90SH-400 from Shin Etsu. The cellulose derivative may also be cellulose sulfate (Ushercell).

The pH of the pharmaceutical composition may be from 5.7 to 6.7. The pH of the composition may be from 6.0 to 6.5 or from 6.0 to 6.3. Preferably the pH of the pharmaceutical composition is from 6.0 to 6.2.

Typically the pharmaceutical product is in the form of a hydrogel. Hydrogels comprise a crosslinked network of hydrophilic polymers, such as cellulose ethers. Hydrogels possess the ability to absorb a large amount of water and swell, while maintaining their three-dimensional structure.

The pharmaceutical composition may be a vaginal pessary, typically a vaginal hydrogel pessary. In alternative embodiments the pharmaceutical composition may be a tablet, liquid suspension or dispersion, dried powder, topical ointment, cream, foam, gel, polymeric nanoparticles, bioadhesive polymers, fast dissolving film, suppository, or aerosol. The pharmaceutical composition may be applied to a base material, for example a cotton or gauze material, such as a tampon.

The pharmaceutical composition may be administered directly to the vagina or can be administered using a solid device, for example an applicator.

The pharmaceutical composition may be mucoadhesive. Traditional commercial preparations for treating vaginal conditions are known to reside in the vaginal cavity for a relatively short period of time owing to the self-cleaning action of the vaginal tract, and often require multiple daily doses to ensure the desired therapeutic effect. The composition of the present invention is preferably mucoadhesive, i.e. it is capable of adhering to the vaginal mucosa. The mucoadhesive nature of the composition prolongs the residence time of the pharmaceutical composition in the vaginal cavity. Cellulose ethers, such as HPMC are typically mucoadhesive polymers.

The pharmaceutical composition for use of the invention may comprise of or consist of a mucoadhesive film, gel or mucoadhesive *in situ* gelling liquid crystalline precursor system.

The pharmaceutical composition for use may further comprise one or more pharmaceutically acceptable excipients. Pharmaceutically acceptable excipients that can be included in the pharmaceutical compositions of the present invention include, for example, physiologically tolerable surfactants, solvents, emollients, colorants, fragrances, gelling agents, emulsifiers, buffering agents, binders, antioixidants, disintegrants, suspending agent, chelating agent and the like, which are well known in the art. Possible excipients are set out in Table II of "Compendium of Pharmaceutical Excipients for Vaginal Formulations", S. Garg et al, Pharmaceutical Technology Drug Delivery 2001.

The pharmaceutical composition may be free from preservatives, for example the composition may comprise less than 0.1 w/v % preservative.

The pharmaceutical composition may further comprise one or more additional therapeutic agents. The one or more additional therapeutic agents may be selected from an antibiotic, an antibacterial agent, an antifungal agent, an antiparasitic agent, an antiviral agent, an antiseptic agent and an anti-inflammatory agent.

The one or more additional therapeutic agents may be selected from:
antibacterial agents such as C31G, trimethoprim, sulfamethoxazole, and chloromycetin;
antiseptic agents such as chlorhexidine gluconate;
antibiotic agents such as erythromycin, penicillins, cephalosporins and their derivatives, ampicillin, methicillin, and doxycycline;
anti-inflammatory agents such as naproxen, indomethacin, and hydrocortisone;
antiparasitic agents such as thiabendazole;
antiprotozoal agents such as metronidazole, and chloroquine hydrochloride;
antiviral agents such as dextran sulfate and other sulfated polysaccharides, squalamine, and vidarabine;
and antifungal agents such as ketoconazole, flucytosine, itraconazole, amphotericin B, nystatin, butoconazole nitrate, and clotrimazole.

In a further aspect, the present invention provides a method for the prevention or treatment of a condition associated with a reduction in the number of Lactobacilli in the vagina of a subject relative to a healthy subject, the method comprising the step of administering to a subject in need thereof a pharmaceutical composition as defined above.

The subject may be a human or non-human animal.

In an embodiment of the pharmaceutical composition for use or method of the invention the use or method comprises providing an increase in the number of Lactobacilli in the vagina of the subject relative to an untreated subject with the condition. In one embodiment the increase in the number of Lactobacilli is achieved for a period of at least 48 hours, optionally 72 hrs. In an embodiment of the pharmaceutical composition for use or method of the invention the use or method comprises restoring the number of Lactobacilli in the vagina to a normal level, i.e. restoring the number of Lactobacilli in the vagina of the subject to a number of Lactobacilli found in a healthy subject. The number of Lactobacilli in a healthy subject is patient dependent, but a healthy premenopausal woman may have 10⁷-10⁸ CFU Lactobacilli mL⁻¹ vaginal fluid.

Methods of measuring the number of lactobacilli in the vagina are discussed above and would be known to a person skilled in the art. For example, a swab may be taken from the vagina of the subject, and used to inoculate a medium suitable for growth of Lactobacilli. After incubation, the grown bacteria are then cultured onto Lactobacilli specific plates, and incubated for a suitable period of time. The number of bacterial colonies is then counted. The number of Lactobacilli is typically expressed in colony-forming units (CFU).The Lactobacilli may be endogenous Lactobacilli, i.e. normally present in the vagina of a subject who is healthy. It will be understood that the vaginal microflora can vary significantly between individuals, and there may be significant variation in the number and type of Lactobacilli and other microorganisms present in the vaginal cavity between individuals. However, the Lactobacilli may comprise *Lactobacillus gasseri, Lactobacillus acidophilus, Lactobacillus paracasei, Lactobacillus salivarius, Lactobacillus johnsonii* or combinations thereof.

The condition to be prevented or treated is typically a vaginal infection, vaginal inflammation, or complications therefrom. The condition may be a recurrent vaginal infection or vaginal inflammation. A recurrent vaginal infection or inflammation is one in which symptoms recur despite initial reduction or elimination of symptoms, for example symptoms of infection or inflammation recur after one, two or three months. An infection may recur because although a population of pathogenic bacteria is significantly reduced after an initial treatment, allowing regrowth of the beneficial bacteria such as Lactobacilli, the pathogenic bacteria will not be completely eliminated and will regrow over time, causing a recurrence of the infection.

The composition of the invention is particularly suitable for treating recurrent infections because it is understood to support the regrowth of any Lactobacilli, not just those which are acid tolerant such as those found after treatment with the lactic-acid containing pharmaceutical compositions of the prior art. By supporting regrowth of a broader range of Lactobacilli, the regrowth of pathogenic bacteria or yeast is less likely, preventing recurrence of an infection such as BV or vulvovaginal candidiasis.

The pharmaceutical composition may be used to prevent or treat complications of the vaginal infection or vaginal inflammation such as preterm labour or birth, stillbirth, postpartum vaginal infection or vaginal inflammation. It is well known that vaginal infections such as BV provide an increased risk of preterm labour, birth, stillbirth, first-trimester miscarriage, optionally in women undergoing IVF, amniotic-fluid infection, chorioamnionitis, endometritis after childbirth or abortion, infections after hysterectomy, pelvic inflammatory disease, and postpartum infection/inflammation (see for example Romero, R. et al. (2001). "The role of infection in preterm labour and delivery," Journal of Pediatric and Perinatal Epidemiology, 15(2), pp.41-56., Warr AJ, et al, "Sexually transmitted infections during pregnancy and subsequent risk of stillbirth and infant mortality in Kenya: a prospective study", BMJ 2018;0:1-7. doi:10.1136/sextrans-2018-053597, Baqui et al, "Prevalence of and risk factors for abnormal vaginal flora and its association with adverse pregnancy outcomes in a rural district in north-east Bangladesh", Acta Obstet Gynecol Scand. 2018;1-11, and Paavonen, J. et al, "Bacterial Vaginosis and Desquamative Inflammatory Vaginitis", N Engl J Med 2018; 379:2246-2254).

In preferred embodiments of the invention the condition to be prevented or treated is bacterial vaginosis, vaginitis, vulvovaginal candidiasis or Group B *Streptococcus* infection.

In embodiments of the composition for use or method of the invention the condition to be prevented or treated is associated with an increase in the number of pathogenic bacteria, optionally *Gardnella vaginalis* bacteria or yeast in the vagina of a subject relative to a healthy subject. Pathogenic bacteria or yeast are any bacteria or yeast which can cause a disease or medical condition.

Typically the pharmaceutical composition is suitable for intravaginal administration. The pharmaceutical composition may be administered whilst the patient is supine, typically at night time.

In an additional aspect, the present invention provides a method of preparing a pharmaceutical composition, the method comprising:
(a) heating water to about 80 °C
(b) gradually adding a cellulose derivative to the heated water under agitation in an amount to provide an aqueous solution comprising 2 to 5 w/v % of the cellulose derivative
(c) cooling the aqueous solution to form a gel
(d) separately providing an aqueous solution of glycogen at 1 to 5 °C;
(e) mixing the gel with the aqueous solution of glycogen to provide the pharmaceutical composition, wherein the pharmaceutical composition comprises 1.3 to 1.7 w/v % glycogen, optionally 1.4 to 1.6 w/v %, optionally about 1.5 w/v %.

The cellulose derivative may be any cellulose derivative described above, for example HPMC. Agitation is typically achieved by stirring the solution, for example with a magnetic stirrer. In step (e) the mixing is very slow, or gradual mixing of the gel with the aqueous solution of glycogen. The direct product of the method is a preferred embodiment of the composition used in the pharmaceutical composition for use in a method of treatment of the invention. The invention will now be further described by way of Examples, which are meant to serve to assist one of ordinary skill in the art in carrying out the invention and are not intended in any way to limit the scope of the invention.

### Example 1

This study was based on a within subject variation, therefore, only one patient was enrolled to the study after completion of screening questionnaires and giving their consent. The patient was diagnosed with BV using three of the Amsel's criteria (positive whiff test, grayish white homogeneous discharge and presence of clue cells). This method is well known to a person skilled in the art. A swab was taken from the patient and inoculated in artificial vaginal fluid (AVF) where the carbon source was replaced with glycogen.

### Gel formulation

The gel was prepared containing the following concentrations of glycogen, 0, 0.5, 1.0, 1.5 and 5% w/v in 100ml glass media bottles. Both cold and hot processes of gel making were utilized in this method where 60mL of water was heated to 80°C to which 2.5g of the semisynthetic polymer, hydroxypropyl methylcellulose (HPMC) (Metolose^{®} 90SH-400) was gradually added whilst the solution was continually being stirred on a magnetic hot-plate stirrer. Glycogen was added to a 20mL volumetric flask in the relative quantities and made up with cold water, also being placed on a magnetic stirrer. The formulated gel was then mixed and allowed to cool to room temperature. The dissolved glycogen was then added to the gel sample and the remaining 20mL of cold water also added gradually. All samples were then labelled, autoclaved and placed in the fridge.

### Bacterial growth media preparation

3L (144 agars) of the Rogosa Agar was prepared by adding 20.5g of the growth media into 6 x 500mL glass media bottles to which 1.3mL of glacial acetic acid was added, before making it up to 250mL with distilled water. The samples were then placed in the autoclave before being poured into the plates under sterile conditions.

### Preparation of the Artificial Vaginal Fluid

Two Artificial Vaginal Fluids (AVFs) were prepared, one containing carbon (control) and one not. 300mL of the AVF not containing carbon was prepared by the addition of the relative amounts of ingredients shown in table 1, and making it up to 300mL with distilled water. The same procedure was carried out for the second AVF, but in 100mL with the addition of the carbon amount stated in Table 1, before placing both fluids into the autoclave. Within a sterile fume hood, 10mL of the control AVF was added to eight Sterilin specimen tubes, labelled and placed in the fridge. 5mL of the second AVF was then also added to eight different tubes for each concentration of glycogen including the blank before adding 5mL of the corresponding concentration of the prepared gels, labelling and placing in the fridge.

| **Table 1. Relative amounts of ingredients used to prepare both AVFs** | | |
|---|---|---|
| **Ingredient** | **Amount (g) per 300mL** | **Amount (g) per 100mL** |
| **Peptone** | 1.5 | 0.5 |
| **Yeast extract** | 0.75 | 0.25 |
| **Sodium acetate** | 0.75 | 0.25 |
| **Dipotassium phosphate** | 0.3 | 0.1 |
| **Ammonium sulphate** | 0.3 | 0.1 |
| **Magnesium sulphate** | 0.03 | 0.01 |
| **Carbon source** | - | 1.5 |

### Sub-culturing bacterial samples

Swabs retrieved from patients positively diagnosed with BV by using three of the Amsel's criteria (positive whiff test, grayish white homogeneous discharge and presence of clue cells)were incubated in a broth containing a 1.5% w/v of glycogen and glucose for 3-4 days anaerobically at 37°C to encourage the growth of the *Lactobacilli* bacterial strains. A 10µL sample was then inoculated into three plates specific for *Lactobacilli* and incubated for a further 3-4 days anaerobically.

### Sub-culturing into gel samples, plating and colony counting

Different colonies from the grown bacterial colonies were inoculated into each of the control broths containing carbon using a 10µL inoculation loop and incubated for 3 days anaerobically. From the grown bacteria, 10µL was then inoculated into all 5 gel sample broths including the blank and further incubated for 4 days. The grown bacteria were then cultured onto *Lactobacilli* specific plates set in triplicates for each gel concentration and incubated for 3 days anaerobically before being counted.

### Bacterial colonv counts

All grown bacterial colonies were counted on a Stuart SC6 colony counter. Figure 1 shows the average of the bacterial counts for each gel glycogen concentration, attained from the eight colonies inoculated from the patient swabs. It is apparent that the glycogen concentration with the highest bacterial count is 1.5% w/v; this result is consistent with all eight colonies. The standard deviation error bars also show the deviation of each bacterial count from mean value (true value).

An analysis of variance (ANOVA) test was carried out to determine the deviation between the counts and whether the variable (gel concentration) has an effect on the bacterial counts or not. Where the f-value is greater than the f-critical value, the null hypothesis is rejected. The null hypothesis being that the variable has an effect on the bacterial colony count. When the null hypothesis is rejected, the alternative hypothesis must be accepted which is that the variable does not have an effect on the colony count. It was found that for colonies one, two, three and six, the f-critical value was lower than the f-value, rejecting the null hypothesis and accepting the alternative that the gel concentration does not have an effect on the colony count. On the other hand, colonies four, five, seven, and eight, all accepted the null hypothesis due to the f-critical value being higher than the f-value, suggesting there is an evident correlation between the variable and the colony count.

### Example 2

The study was repeated with 2 further patients, the results of which are shown in Figures 2 and 3. The same protocol was used for patients 2 and 3 as for patient 1, with the exception that three colonies rather than eight colonies from each patient were grown, and the inoculated control broth was incubated for a shorter period of time (2 days rather than 3 days). In addition, the formulations tested contained 0, 0.5, 1, 1.5. 2 and 2.5 w/v % glycogen. To summarise, different colonies from the grown bacterial colonies were inoculated into each of the control broths containing carbon using a 10µL inoculation loop and incubated for 2 days (not 3 days as in Example 1) anaerobically. From the grown bacteria, 10µL was then inoculated into all 5 gel sample broths including the blank and further incubated for 4 days. The grown bacteria were then cultured onto *Lactobacilli* specific plates set in triplicates for each gel concentration and incubated for 3- days anaerobically before being counted.

Examples 1 and 2 demonstrate that that the composition of the invention comprising glycogen has the ability to restore the vaginal flora, with the composition comprising 1.5% w/v glycogen achieving the highest counts of the bacterial species that typically dominates the vaginal environment in healthy women, *Lactobacillus.* The restoration of this bacterial species is associated with the treatment of BV and therefore compositions of the invention comprising from 1.3 to 1.7 w/v% glycogen, optimally 1.5 w/v % glycogen, can provide an efficacious non-antibiotic treatment of BV and other vaginal infections and inflammations. It can be seen from Figures 1 to 3 that compositions of the invention comprising 1.5 w/v % glycogen provide a much higher Lactobacillus count than compositions outside the scope of the claim comprising 0.5, 1.0, 2.0, 2.5 or 5.0 w/v % glycogen. Without being bound by theory it is understood that 1.5 w/v % glycogen provides an optimum concentration of glycogen such that it acts as a carbon source for Lactobacilli, aiding their growth. At lower concentrations, for example 1 w/v % glycogen, the concentration of carbon is not sufficient to support good Lactobacillus growth. Higher concentrations, e.g. 2 w/v % glycogen, or 2.5 to 17 w/v% as disclosed in EP1072269 can actually inhibit bacterial growth.

### Example 3 - Comparison with Commercially available composition (Canesbalance ^{®} BV gel)

### Materials & Methods

Microbiological assessment of pessary performance *in vitro:* Two vaginal swabs were taken from opposite sides of the vagina of each patient. The two vaginal swabs were then inoculated into either 10 mL Vaginal Simulating Fluid or, 5 mL Vaginal Simulating Fluid where the carbon source had been replaced by 5 mL of the pessary composition of the invention (gel formulation of Example 1 comprising 1.5 % w/v glycogen). Vaginal Simulating Fluid or Artificial Vaginal Fluid has been previously described in D.H. Owen and D.F. Katz, "A Vaginal Fluid Simulant", Contraception, 1999; 59; 91-95, and M. S. J. Tomas and M. E. Nader-Macias, "Effect of a medium simulating vaginal fluid on the growth and expression of beneficial characteristics of potentially probiotic lactobacilli", Communicating Current Research and Educational Topics and Trends in Applied Microbiology, 2007.

The Vaginal Simulating Fluid (or Artificial Vaginal Fluid) contained glucose (adjusted to a final concentration of 1.5%), peptone (10.0), yeast extract (5.0), sodium acetate (5.0), dipotassium phosphate (2.0), ammonium sulphate (2.0), magnesium sulphate (0.2), manganese sulphate (0.1), Tween 80 (1 mL), pH 4.5. Both broths were incubated anaerobically and without agitation at 37 °C for 72 hrs. The broths were then sub-cultured onto Lactobacillus Selection Agar (Rogosa agar). After 96 hrs anaerobic incubation at 37 °C, the number of colonies visible on the agar plates was compared. Only lactobacilli can grow on the Rogosa agar and this was confirmed by light microscopy of Gram stained colonies, which gave morphology consistent with lactobacilli. The numbers of bacteria recovered after initial incubation in Vaginal Simulating Fluid will give an indication of the numbers of residual lactobacilli present during active BV disease. We were expecting that the numbers of bacteria recovered after initial incubation in Vaginal Simulating Fluid where the carbon source was replaced by the pessary, would show a shift towards increased numbers of *Lactobacillus* spp., if the glycogen was performing as a prebiotic in the pessary formulation.

### Results

Table 2 below shows the number of bacteria recovered after 96 hours incubation on media specific for lactobacilli following pre-treatment of swabs for 72 hours in Artificial Vaginal Fluid, our formulation or the market brand leader Canesbalance^{®}.

The Artificial Vaginal Fluid contains glucose as the sole carbon source and so should allow for all types of bacteria sampled from the patient to grow, including lactobacilli. Our formulation contains glycogen as the sole carbon source and should only encourage regrowth of the residual lactobacilli population, if this population still exists in the diseased vaginal flora. Canesbalance^{®} contains glycogen (and lactic acid) and should, like our formulation; act as a prebiotic to encourage regrowth of residual lactobacilli.

The results show that there were significantly (p<0.05) more lactobacilli recovered following pre-incubation of the swabs in the glycogen only formulation of the invention compared to the Artificial Vaginal Fluid and, surprisingly, Canesbalance^{®}. This suggests that the formulation of the invention can encourage significant regrowth of the lactobacilli that remain during a disease episode. The minor variation in the results between patients was as expected from a randomly selected patient population, suggesting that the majority of patients would respond in the same way if our formulation were used as a treatment.

**Table 2**

| | **Mean number of lactobacilli n=3 (10⁴ xCFU mL⁻¹)** | |
|---|---|---|
| **Patient number** | **Artificial Vaginal Fluid** | **Glycogen only formulation** |
| **1** | 0.2 | 3.6 |
| **2** | 0.6 | 3.7 |
| **3** | 0 | 1.1 |
| **4** | 0 | 1.1 |
| **Average** | 0.2 | 2.4 |
| | | |

| | **Canesbalance^{®}** | **Glycogen only formulation** |
|---|---|---|
| | | |
| 5 | 0 | 3.3 |
| 6 | 0 | 1.2 |
| 7 | 0 | 3.2 |
| 8 | 0 | 0 |
| 9 | 0 | 0.05 |
| 10 | 0 | 0 |
| 11 | 0 | 0 |
| 12 | 0 | 3.8 |
| 13 | 0 | 1.2 |
| 14 | 0 | 0 |
| 15 | 0 | 1.4 |
| 16 | 0 | 4.2 |
| 17 | 0 | 10.4 |
| 18 | 0 | 9 |
| **Average** | 0 | 2.7 |

### Example 4 - Addition of lactic acid inhibits lactobacillus growth

A gel formulation was prepared according to Example 1 comprising 1.5 w/v% glycogen. A comparative gel formulation was prepared according to Example 1 comprising 1.5 w/v% glycogen, but with the addition of lactic acid at a concentration of 225 mg per 5 mL. The comparative formulation had a pH of 3.5. The comparative formulation had the same lactic acid concentration and pH as commercial product Canesbalance^{®}. Table 3 demonstrates that the addition of lactic acid to the formulation of the invention inhibits lactobacillus growth.

| **Table 3** | **Mean number of lactobacilli n=3 (10⁴ x CFU mL⁻¹)** | |
|---|---|---|
| **Patient Number** | **Glycogen only formulation (invention)** | **Glycogen only formulation + lactic acid** |
| 21 | 2.9 | 0.3 |
| 22 | 2.3 | 0 |
| 23 | 3.9 | 0 |

### Example 5 - Bacterial carbon source

Swabs were incubated in either in Vaginal Simulating Fluid or Vaginal Simulating Fluid in combination with the gel formulation of the invention (1.5 w/v% glycogen), and broths were plated onto blood agar and incubated aerobically for 72 hrs at 37 °C (blood agar will encourage most bacteria, including BV associated bacteria; aerobic conditions were used because lactobacilli are unlikely to grow but any colonies showing signs of alpha haemolysis from were excluded from the counts as potential lactobacilli). If the bacterial counts were higher for the formulation of the invention than the control, this would suggest that other bacteria were using the formulation of the invention as a carbon source and out competing lactobacilli (i.e. not being inhibited by the lactobacilli). This was not the case, and Table 4 below shows that the mean number of bacteria was lower in the presence of the gel formulation of the invention than in the control, meaning that bacteria other than lactobacilli were not using the formulation of the invention as a carbon source.

**Table 4**

| **Blood agar** | **Mean n=3 (10⁴ x CFU mL⁻¹)** | |
|---|---|---|
| **Patient number** | **Control** | **Glycogen only formulation (invention)** |
| 24 | 2.7 | 0.7 |
| 25 | 4.1 | 3.7 |

### Example 6 - Physico-chemical parameters indicative of formulation of the invention encouraging regrowth of lactobacilli

Table 5 indicates that the administration of the gel formulation of the invention containing 1.5 w/v% glycogen leads to improvements in vaginal pH, lactic acid concentration and hydrogen peroxide concentration relative to administration of a control formulation.

**Table 5**

| **Patient number** | **pH (> 4.5 indicates BV)** | | **D-lactic acid concentration mM (healthy ~ 100 mM)** | | **Hydrogen peroxide concentration µM (healthy ~ 100 µM)** | |
|---|---|---|---|---|---|---|
| | **Control** | **Glycogen only formulation (invention)** | **Control** | **Glycogen only formulation (invention)** | **Control** | **Glycogen only formulation (invention)** |
| 24 | 5.5 | 4.2 | 0 | 45 | 0 | 0 |
| 25 | 5.2 | 3.8 | 22.5 | 90 | 0 | 88 |

### Example 7 - Alteration of vaginal bacterial population

Figure 4 shows a pie chart of the relative proportions of bacterial species in the vagina of a patient (A) before treatment with the composition of the invention and (B) after treatment with the composition of the invention as determined by amplicon sequencing the V1/V2 hypervariable region of bacterial 16 S rRNA gene (Ravel J, et al. Vaginal microbiome of reproductive-age women (Proc Natl Acad Sci USA. 2011;108:4680-4687. doi: 10.1073/pnas.1002611107). It can be seen that the proportion of Lactobacilli increases from 71.4% (pre-treatment) to 85.4% (post-treatment). The proportion of Enterococci decreases from 24.7% (pre-treatment) to 12.9 % (post-treatment).

### Example 8 - Treatment of group B Streptococcus infection in pregnant patients

Pregnant patients known to be colonised by Group B streptococci were recruited. The method for detection of Group B Streptococci carriage followed UK Standards for Microbiology Investigations, Public Health England: Bacteriology B 58 Issue no: 3.1 Issue date: 26.06.18 - as shown in Table 6 below.

Results of culture dependent colony counts of Group B streptococci (GBS) from swabs incubated in the LIM control broth vs LIM broth plus 1.5 % w/v glycogen are provided in Table 7. Colonies were counted after incubation on chromogenic agar and results from 10 of the 12 patients show a significant (p≤ 0.05) reduction in numbers from swabs incubated in the formulation of the invention comprising 1.5 % w/v glycogen, providing evidence that there would be a reduction in carriage of GBS in pregnant women if the formulation of the invention is used as a treatment.

**Table 6**

| **Clinical details/ Conditions** | **Specimen** | **Standard media** | **Incubation** | | | **Cultures read** | **Target organism(s)** |
|---|---|---|---|---|---|---|---|
| | | | **Temp °C** | **Atmos** | **Time** | | |
| Carriage of Group B streptococci | Maternal low vaginal and anorectal swabs | LIM Broth (5mL)^{†}: Todd-Hewitt broth supplemented with 10µg/mL colistin - or 8µg/mL gentamicin and 15µg/mL nalidixic acid | 35-37 | 5% CO₂ | 18-24hr | N/A | |
| | | Then subculture to: | 35-37 | 5% CO2 | 24-48hr | 18-24hr | Group B streptococci |
| | | Blood agar | | | | and | |
| | | or | | | | 48hr | |
| | | Selective agar | 35-37 | Ambient | 24-48hr | 18-24hr | |
| | | or | | | | | |
| | | Chromogenic agar | 35-37 | Ambient | 24-48hr | 18-24hr | |
| †The bottle should contain a volume of broth sufficient to cover the swabs | | | | | | | |

| **Table 7** | **Mean number of GBS colonies n=9 (10⁴ x CFU mL⁻¹)** | |
|---|---|---|
| **Patient No.** | **Control** | **Formulation of invention** |
| 1 | 4.7 | 0.29 |
| 2 | 2.7 | 0.6 |
| 3 | 4.9 | 2.2 |
| 4 | 0.85 | 0.4 |
| 5 | 0.46 | 0.11 |
| 6 | 5.22 | 2.35 |
| 7 | 4.39 | 1.83 |
| 8 | 2.8 | 1.74 |
| 9 | 4.5 | 1.99 |
| 10 | 4.78 | 1.25 |
| 11 | 0.66 | 0.41 |
| 12 | 2.23 | 2.14 |

For each of patients 1 to 10, P <0.05.

### References

(1) Eschenbach D A, Gravett M G, Chen K C, Hoyme U B, Holmes K K. Bacterial vaginosis during pregnancy. An association with prematurity and postpartum complications. Scand J Urol Nephrol Suppl. 1984; 86:213-222.
(2) Hillier S L, Nugent R P, Eschenbach D A, et al. Association between bacterial vaginosis and preterm delivery of a low-birth-weight infant. The Vaginal Infections and Prematurity Study Group. N Engl J Med. 1995; 333(26):1737-1742.
(3) Watts D H, Eschenbach D A, Kenny G E. Early postpartum endometritis: the role of bacteria, genital mycoplasmas, and Chlamydia trachomatis. Obstet Gynecol. January 1989; 73(1):52-60.
(4) Martin H L, Richardson B A, Nyange P M, et al. Vaginal lactobacilli, microbial flora, and risk of human immunodeficiency virus type 1 and sexually transmitted disease acquisition. J Infect Dis. December 1999; 180(6):1863-1868.
(5) Koumans E H, Kendrick J S. Preventing adverse sequelae of bacterial vaginosis: a public health program and research agenda. Sex Transm Dis. May 2001; 28(5):292-297.
(6) Witkin, S. S., I. M. Linhares, P. Giraldo, and W. J. Ledger. 2007. An altered immunity hypothesis for the development of symptomatic bacterial vaginosis. Clin Infect Dis 44:554-7.
(7) Fredricks D N, Fiedler T L, Thomas K K, Mitchell C M, Marrazzo J M. Changes in Vaginal Bacterial Concentrations with Intravaginal Metronidazole Therapy for Bacterial Vaginosis as Assessed by Quantitative PCR. J Clin Microbiol. Jan. 14, 2009.
(8) Josey W E, Schwebke J R. The polymicrobial hypothesis of bacterial vaginosis causation: a reassessment. Int J STD AIDS. March 2008; 19(3):152-154.
(9) Schwebke J R, Rivers C, Lee J. Prevalence of Gardnerella vaginalis in Male Sexual Partners of Women With and Without Bacterial Vaginosis. Sex Transm Dis. Sep. 10, 2008.
(10) Gardner H L, Dukes C D. Haemophilus vaginalis vaginitis: a newly defined specific infection previously classified non-specific vaginitis. Am J Obstet Gynecol. May 1955; 69(5):962-976.
(11) Aroutcheva, A. A., J. A. Simoes, K. Behbakht, and S. Faro. 2001. Gardnerella vaginalis isolated from patients with bacterial vaginosis and from patients with healthy vaginal ecosystems. Clin Infect Dis 33:1022-7.
(12) Rosenstein, I. J., et al, Relationship between hydrogen peroxide producing strains of lactobacilli and vaginosis associated with bacterial species in pregnant women, Eur. J. Clin. Microbial. Infect. Dis. 1997, 6:517-22.
(13) Amsel, R., et al., Nonspecific vaginitis. Diagnostic criteria and microbial and epidemiologic associations, Am. J. Med 1983, 74:14-22;
(14) Larsson, P. G., Treatment for bacterial vaginosis: an update on the expected cure rate, Int'l J. of STD & AIDS 1997, 8:35-6.
(15) Larsson, P. G. Treatment of bacterial vaginosis. Int. J. STD AIDS 1992; 3: 239-247.
(16) Wilson, 2004, Sex Transm Infect 80:8-11.
(17) See, Hillier et al., June 1993, "Efficacy of Intravaginal 0.75% Metronidazole Gel for the Treatment of Bacterial Vaginosis," Obstet Gynecol 81(6):963-967
(18) Scholes, D., et al., Vaginal douching as a risk factor for acute pelvic inflammatory disease, Obstet. Gynecol. 1993, 81:601-6;
(19) Darling J. R., et al., Vaginal douching and the risk of tubal pregnancy, Epidemiology 1991, 2:40-8;
(20) Kendrick, J. S., et al., Vaginal douching and the risk of ectopic pregnancy among black women, Am. J. Obstet. Gynecol. 1997, 176:991-7;
(21) Baird, D. D., et al., Vaginal Douching and reduced fertility, Am. J Public Health 1996, 86:844-50.
(22) Warr AJ, et al. Sexually transmitted infections during pregnancy and subsequent risk of stillbirth and infant mortality in Kenya: a prospective study. BMJ 2018;0:1-7. doi:10.1136/sextrans-2018-053597
(23) Baqui et al. Prevalence of and risk factors for abnormal vaginal flora and its association with adverse pregnancy outcomes in a rural district in north-east Bangladesh Acta Obstet Gynecol Scand. 2018;1-11.
(24) Bianchi-Jassir et al Preterm Birth Associated With GBS Maternal Colonization worldwide: Systematic review and meta -analyses. CID 2017:65 (Suppl 2)
(25) Lin FY, Weisman LE, Troendle J, Adams K. Prematurity is the major risk factor for late-onset group B Streptococcus disease. J Infect Dis 2003; 188:267-71.
(26) Proc Natl Acad Sci USA. 2011;108:4680-4687
(27) B. Vitali. C. Pugliese, E. Biagi, M. Candela, S. Turroni, G. Bellen, G. G. G. Donders, P. Brigidi, Dynamics of vaginal bacterial communities in women developing bacterial vaginosis, candidiasis, or no infection, analyzed by PCR-denaturing gradient gel electrophoresis and real-time PCR. Appl. Environ. Microb, 2020, 73(18), 5731-5741.
(28) V. D. Mijac, S. V. Dukic, N. Z. Opavski, M. K. Dukic, L. T. Ranin, Hydrogen peroxide producing lactobacilli in women with vaginal infections. Eur. J. Obstet. Gyn. R. B 2006, 129(1), 69-76.
(29) E. Biagi, B. Vitali, C. Pugliese, M, Candela, G.G.G. Donders, P. Brigidi, Quantitative variations in the vaginal bacterial population associated with asymptomatic infections: a real-time polymerase chain reaction study. Eur. J. Clin. Microbiol. 2009, 28, 281-285.
(30) L. Akimoto-Gunther, P. de Souza Bonfirm-Mendonca, G. Takahachi, M. M. T. Irie, S. Miyamoto, M. E. Lopes Consolaro, T. I. E. Svidzinsk, Highlights regarding host predisposing factors to recurrent vulvovaginal candidiasis: chronic stress and reduced antioxidant capacity. PLoS One 2016, 11(7), e0158870. DOI:10.1371/journal.pone.0158870.
(31) C. Ceccarani, C. Foschi, C. Parolin, A. D'Antuono, V. Gaspari, C. Consolandi, L. Laghi, T. Camboni, B. Vitali, M. Severgnini, A. Marangoni, Diversity of vaginal microbiome and metabolome during genital infections. Sci. Rep. 2019, 9, 14095. DOI:10.1038/s41598-019-50410-x.
(32) X. Zhou, R. Westman, R. Hickey, M. A. Hansmann, C. Kennedy, T. W. Osborn, L. J. Forney, Vaginal microbiota of women with frequent vulvovaginal candidiasis. Infect. Immun 2009, 77(9), 4130-4135. DOI:10.1128/IAI.00436-09.
(33) Allonsius, C.N., Vandenheuvel, D., Oerlemans, E.F.M. et al. Inhibition of Candida albicans morphogenesis by chitinase from Lactobacillus rhamnosus GG. Sci Rep 9, 2900 (2019). https://doi.org/10.1038/s41598-019-39625-0.

## Claims

1. A pharmaceutical composition for use in the prevention or treatment of a condition associated with a reduction in the number of Lactobacilli in the vagina of a subject relative to a healthy subject, wherein the composition comprises from 1.3 to 1.7 w/v % glycogen and less than 0.1 w/v % lactic acid, optionally wherein the composition contains no lactic acid.

2. A pharmaceutical composition according to claim 1, further comprising water and a cellulose derivative, optionally wherein the composition comprises from 94 to 97 w/v % water and from 2 to 5 w/v % of a cellulose derivative, wherein the cellulose derivative is any polymer comprising cellulose.

3. A pharmaceutical composition according to any preceding claim for the use according to any preceding claim wherein the composition comprises an aqueous hydrogel comprising a cellulose derivative, wherein the cellulose derivative is any polymer comprising cellulose.

4. A pharmaceutical composition according to any preceding claim for the use according to any preceding claim wherein the composition comprises from 1.4 to 1.6 w/v % glycogen, optionally about 1.5 w/v % glycogen, optionally wherein the glycogen is obtainable from an animal source, further optionally wherein the glycogen is oyster, mussel or bovine glycogen.

5. A pharmaceutical composition according to any of claims 2 to 4 for the use according to any of claims 2 to 4 wherein the cellulose derivative is a cellulose ether, optionally wherein the cellulose ether is methylcellulose (MC), hydroxypropyl methylcellulose (HPMC), ethyl cellulose (EC), hydroxyethyl cellulose (HEC), sodium carboxymethylcellulose (NaCMC) , or combinations thereof, further optionally wherein the cellulose ether is hydroxypropyl methylcellulose.

6. A pharmaceutical composition according to any preceding claim for the use according to any preceding claim wherein the pH of the composition is from 5.7 to 6.7, optionally wherein the pH of the composition is from 6.0 to 6.2.

7. A pharmaceutical composition according to any preceding claim for the use according to any preceding claim which is in the form of a hydrogel and/or a vaginal pessary.

8. A pharmaceutical composition according to any preceding claim for the use according to any preceding claim which is mucoadhesive, optionally wherein the composition comprises a mucoadhesive film, gel or mucoadhesive *in situ* gelling liquid crystalline precursor system.

9. A pharmaceutical composition according to any preceding claim further comprising one or more pharmaceutically acceptable excipients and/or further comprising one or more additional therapeutic agents, optionally wherein the one or more additional therapeutic agents are selected from an antibiotic, an antibacterial agent, an antifungal agent, an anti-parasitic agent, an antiviral agent, antiseptic agent and an anti-inflammatory agent.

10. A pharmaceutical composition for use according to any preceding claim wherein the use comprises providing an increase in the number of Lactobacilli in the vagina of the subject relative to an untreated subject with the condition, optionally wherein the increase in the number of Lactobacilli is achieved for a period of at least 48 hours and/or wherein the use comprises restoring the number of Lactobacilli in the vagina to a normal level.

11. A pharmaceutical composition for use according to any preceding claim wherein the use comprises attaining a normal pH of the vagina of the subject of from 3.8 to 4.5.

12. A pharmaceutical composition for use according to any preceding claim wherein the condition to be prevented or treated is a vaginal infection, vaginal inflammation, or a complication therefrom, optionally a recurrent vaginal infection or inflammation, further optionally wherein the complication is preterm birth, stillbirth, postpartum vaginal infection or vaginal inflammation, further optionally still wherein the condition to be prevented or treated is bacterial vaginosis, vaginitis, vulvovaginal candidiasis or Group B streptococcus infection.

13. A pharmaceutical composition for use according to any preceding claim wherein the subject to be treated is pregnant.

14. A pharmaceutical composition for use according to any preceding claim wherein the condition to be prevented or treated is associated with an increase in the number of pathogenic bacteria or yeast in the vagina of a subject relative to a healthy subject, optionally Gardnella vaginalis or Group B Steptococcus bacteria or a species of Candida yeast, optionally Candida albicans.

15. A pharmaceutical composition according to any preceding claim for the use according to any preceding claim wherein the composition is suitable for intravaginal administration.

## Patentansprüche

1. Pharmazeutische Zusammensetzung zur Verwendung bei der Prävention oder Behandlung eines mit einer Reduktion der Zahl von Lactobacilli in der Vagina eines Individuums relativ zu einem gesunden Individuum assoziierten Leidens, wobei die Zusammensetzung 1,3 bis 1,7 % w/v Glykogen und weniger als 0,1 % w/v Milchsäure umfasst, gegebenenfalls wobei die Zusammensetzung keine Milchsäure enthält.

2. Pharmazeutische Zusammensetzung nach Anspruch 1, ferner umfassend Wasser und ein Cellulosederivat, gegebenenfalls wobei die Zusammensetzung 94 bis 97 % w/v Wasser und 2 bis 5 % w/v eines Cellulosederivats umfasst, wobei es sich bei dem Cellulosederivat um ein beliebiges Polymer, das Cellulose umfasst, handelt.

3. Pharmazeutische Zusammensetzung nach einem der vorhergehenden Ansprüche zur Verwendung nach einem der vorhergehenden Ansprüche, wobei die Zusammensetzung ein wässriges Hydrogel umfasst, welches ein Cellulosederivat umfasst, wobei es sich bei dem Cellulosederivat um ein beliebiges Polymer, das Cellulose umfasst, handelt.

4. Pharmazeutische Zusammensetzung nach einem der vorhergehenden Ansprüche zur Verwendung nach einem der vorhergehenden Ansprüche, wobei die Zusammensetzung 1,4 bis 1,6 % w/v Glykogen, gegebenenfalls etwa 1,5 % w/v Glykogen, umfasst, gegebenenfalls wobei das Glykogen aus einer tierischen Quelle erhältlich ist, ferner gegebenenfalls wobei es sich bei dem Glykogen um Austern-, Muschel- oder Rinderglykogen handelt.

5. Pharmazeutische Zusammensetzung nach einem der Ansprüche 2 bis 4 zur Verwendung nach einem der Ansprüche 2 bis 4, wobei es sich bei dem Cellulosederivat um einen Celluloseether handelt, gegebenenfalls wobei es sich bei dem Celluloseether um Methylcellulose (MC), Hydroxypropylmethylcellulose (HPMC), Ethylcellulose (EC), Hydroxyethylcellulose (HEC), Natriumcarboxymethycellulose (NaCMC) oder Kombinationen davon handelt, ferner gegebenenfalls wobei es sich bei dem Celluloseether um Hydroxypropylmethylcellulose handelt.

6. Pharmazeutische Zusammensetzung nach einem der vorhergehenden Ansprüche zur Verwendung nach einem der vorhergehenden Ansprüche, wobei der pH-Wert der Zusammensetzung 5,7 bis 6,7 beträgt, gegebenenfalls wobei der pH-Wert der Zusammensetzung 6,0 bis 6,2 beträgt.

7. Pharmazeutische Zusammensetzung nach einem der vorhergehenden Ansprüche zur Verwendung nach einem der vorhergehenden Ansprüche, die in Form eines Hydrogels und/oder eines Vaginalpessars vorliegt.

8. Pharmazeutische Zusammensetzung nach einem der vorhergehenden Ansprüche zur Verwendung nach einem der vorhergehenden Ansprüche, die mukoadhäsiv ist, gegebenenfalls wobei die Zusammensetzung einen mukoadhäsiven Film, ein mukoadhäsives Gel oder ein mukoadhäsives in situ gelierendes flüssigkristallines Vorläufersystem umfasst.

9. Pharmazeutische Zusammensetzung nach einem der vorhergehenden Ansprüche, ferner umfassend einen oder mehrere pharmazeutisch unbedenkliche Hilfsstoffe und/oder ferner umfassend ein oder mehrere zusätzliche therapeutische Mittel, gegebenenfalls wobei das eine oder die mehreren zusätzlichen therapeutischen Mittel aus einem Antibiotikum, einem antibakteriellen Mittel, einem Antimykotikum, einem Antiparasitikum, einem antiviralen Mittel, einem Antiseptikum und einem entzündungshemmenden Mittel ausgewählt sind.

10. Pharmazeutische Zusammensetzung zur Verwendung nach einem der vorhergehenden Ansprüche, wobei die Verwendung das Bereitstellen einer Erhöhung der Zahl von Lactobacilli in der Vagina des Individuums relativ zu einem unbehandelten Individuum mit dem Leiden umfasst, gegebenenfalls wobei die Erhöhung der Zahl von Lactobacilli über einen Zeitraum von mindestens 48 Stunden erzielt wird und/oder wobei die Verwendung die Wiederherstellung der Zahl von Lactobacilli in der Vagina auf ein normales Niveau umfasst.

11. Pharmazeutische Zusammensetzung zur Verwendung nach einem der vorhergehenden Ansprüche, wobei die Verwendung das Erreichen eines normalen pH-Werts der Vagina des Individuums von 3,8 bis 4,5 umfasst.

12. Pharmazeutische Zusammensetzung zur Verwendung nach einem der vorhergehenden Ansprüche, wobei es sich bei dem zu verhindernden oder zu behandelnden Leiden um eine vaginale Infektion, eine vaginale Entzündung oder eine Komplikation davon, gegebenenfalls eine wiederkehrende vaginale Infektion oder Entzündung, handelt, ferner gegebenenfalls wobei es sich bei der Komplikation um Frühgeburt, Totgeburt, postpartale vaginale Infektion oder vaginale Entzündung handelt, gegebenenfalls des Weiteren wobei es sich bei dem zu verhindernden oder zu behandelnden Leiden um bakterielle Vaginose, Vaginitis, vulvovaginale Candidiasis oder Gruppe-B-Streptokokken-Infektion handelt.

13. Pharmazeutische Zusammensetzung zur Verwendung nach einem der vorhergehenden Ansprüche, wobei das zu behandelnde Individuum schwanger ist.

14. Pharmazeutische Zusammensetzung zur Verwendung nach einem der vorhergehenden Ansprüche, wobei das zu verhindernde oder zu behandelnde Leiden mit einer Erhöhung der Zahl von pathogenen Bakterien oder Hefen in der Vagina eines Individuums relativ zu einem gesunden Individuum, gegebenenfalls Gardnella vaginalis oder Gruppe-B-Streptokokkus-Bakterien oder einer Art von Candida-Hefe, gegebenenfalls Candida albicans, assoziiert ist.

15. Pharmazeutische Zusammensetzung nach einem der vorhergehenden Ansprüche zur Verwendung nach einem der vorhergehenden Ansprüche, wobei die Zusammensetzung für die intravaginale Verabreichung geeignet ist.

## Revendications

1. Composition pharmaceutique destinée à être utilisée dans la prévention ou le traitement d'une affection associée à une réduction du nombre de lactobacilles dans le vagin d'un sujet par rapport à un sujet sain, la composition comprenant de 1,3 à 1,7 % p/v de glycogène et moins de 0,1 % p/v d'acide lactique, la composition ne contenant éventuellement pas d'acide lactique.

2. Composition pharmaceutique selon la revendication 1, comprenant en outre de l'eau et un dérivé de cellulose, la composition comprenant éventuellement de 94 à 97 % p/v d'eau et de 2 à 5 % p/v d'un dérivé de cellulose, le dérivé de cellulose étant un polymère quelconque comprenant de la cellulose.

3. Composition pharmaceutique selon l'une quelconque des revendications précédentes pour l'utilisation selon l'une quelconque des revendications précédentes, la composition comprenant un hydrogel aqueux comprenant un dérivé de cellulose, le dérivé de cellulose étant un polymère quelconque comprenant de la cellulose.

4. Composition pharmaceutique selon l'une quelconque des revendications précédentes, pour l'utilisation selon l'une quelconque des revendications précédentes, la composition comprenant de 1,4 à 1,6 % p/v de glycogène, éventuellement environ 1,5 % p/v de glycogène, le glycogène pouvant éventuellement être obtenu à partir d'une source animale, le glycogène étant éventuellement en outre du glycogène d'huître, de moule ou de bovin.

5. Composition pharmaceutique selon l'une quelconque des revendications 2 à 4, pour l'utilisation selon l'une quelconque des revendications 2 à 4, dans laquelle le dérivé de cellulose est un éther de cellulose, l'éther de cellulose étant éventuellement la méthylcellulose (MC), l'hydroxypropylméthylcellulose (HPMC), l'éthylcellulose (EC), l'hydroxyéthylcellulose (HEC), la carboxyméthylcellulose sodique (NaCMC), ou des combinaisons de celles-ci, l'éther de cellulose étant en outre éventuellement l'hydroxypropylméthylcellulose.

6. Composition pharmaceutique selon l'une quelconque des revendications précédentes, pour l'utilisation selon l'une quelconque des revendications précédentes, le pH de la composition étant compris entre 5,7 et 6,7, le pH de la composition étant éventuellement de 6,0 à 6,2.

7. Composition pharmaceutique selon l'une quelconque des revendications précédentes pour l'utilisation selon l'une quelconque des revendications précédentes, qui est sous la forme d'un hydrogel et/ou d'un pessaire vaginal.

8. Composition pharmaceutique selon l'une quelconque des revendications précédentes, pour l'utilisation selon l'une quelconque des revendications précédentes, qui est mucoadhésive, la composition comprenant éventuellement un film mucoadhésif, un gel ou un système précurseur cristallin liquide gélifiant *in situ* mucoadhésif.

9. Composition pharmaceutique selon l'une quelconque des revendications précédentes, comprenant en outre un ou plusieurs excipients pharmaceutiquement acceptables et/ou comprenant en outre un ou plusieurs agents thérapeutiques supplémentaires, éventuellement dans laquelle les un ou plusieurs agents thérapeutiques supplémentaires sont choisis parmi un antibiotique, un agent antibactérien, un agent antifongique, un agent antiparasitaire, un agent antiviral, un agent antiseptique et un agent anti-inflammatoire.

10. Composition pharmaceutique pour utilisation selon l'une quelconque des revendications précédentes, l'utilisation comprenant une augmentation du nombre de lactobacilles dans le vagin du sujet par rapport à un sujet non traité atteint de l'affection, l'augmentation du nombre de lactobacilles étant éventuellement obtenue pendant une durée d'au moins 48 heures et/ou l'utilisation comprenant le rétablissement du nombre de lactobacilles dans le vagin à un niveau normal.

11. Composition pharmaceutique pour utilisation selon l'une quelconque des revendications précédentes, l'utilisation comprenant l'obtention d'un pH normal du vagin du sujet de 3,8 à 4,5.

12. Composition pharmaceutique pour utilisation selon l'une quelconque des revendications précédentes, dans laquelle l'affection à prévenir ou à traiter est une infection vaginale, une inflammation vaginale ou une complication de celles-ci, éventuellement une infection ou inflammation vaginale récurrente, la complication étant éventuellement en outre une naissance prématurée, une mortinatalité, une infection vaginale post-partum ou une inflammation vaginale, l'affection à prévenir ou à traiter pouvant éventuellement être en outre une vaginose bactérienne, une vaginite, une candidose vulvo-vaginale ou une infection à streptocoques du groupe B.

13. Composition pharmaceutique pour utilisation selon l'une quelconque des revendications précédentes, le sujet à traiter étant enceinte.

14. Composition pharmaceutique pour utilisation selon l'une quelconque des revendications précédentes, l'affection à prévenir ou à traiter étant associée à une augmentation du nombre de bactéries ou levures pathogènes dans le vagin d'un sujet par rapport à un sujet sain, éventuellement des bactéries Gardnella vaginalis ou Steptococcus du groupe B ou une espèce de levure Candida, éventuellement Candida albicans.

15. Composition pharmaceutique selon l'une quelconque des revendications précédentes pour l'utilisation selon l'une quelconque des revendications précédentes, la composition étant adaptée pour l'administration intravaginale.
